# FASCICULE DE BREVET EUROPEEN

(11) **EP 1 317 413 B1**
(45) Date de publication et mention de la délivrance du brevet: **24.08.2005**
(21) Numéro de dépôt: 01955425.2
(22) Date de dépôt: 16.07.2001
(51) Int. Cl.: C07C 233/18, C07C 233/60, C07D 307/81, C07D 333/58, C07D 471/04, C07C 209/16, A61K 31/165, A61K 31/335, A61K 31/38, A61K 31/40

(54) **DERIVES BIPHENYLES SUBSTITUES, LEUR PROCEDE DE PREPARATION ET LES COMPOSITIONS PHARMACEUTIQUES QUI LES CONTIENNENT**
SUBSTITUIERTE BIPHENOLDERIVATE, VERFAHREN ZU IHRER HERSTELLUNG UND DEREN PHARMAZEUTISCHE ZUSAMMENSETZUNGEN
SUBSTITUTED BIPHENYL DERIVATIVES, METHOD FOR PREPARING SAME AND PHARMACEUTICAL COMPOSITIONS CONTAINING SAME

(30) Priorité: 15.09.2000 FR 0011779
(43) Date de publication de la demande: 11.06.2003
(73) Titulaire: Les Laboratoires Servier, 92415 Courbevoie Cedex (FR)
(72) Inventeur: DESCAMPS-FRANCOIS, Carole, F-59260 Hellemmes (FR); YOUS, Said Résidence Marthe Alexandre, F-59120 Loos (FR); LESIEUR, Daniel, F-59147 Gondecourt (FR); GUILLAUMET, Gérald, F-45650 Saint Jean le Blanc (FR); VIAUD, Marie-Claude, F-37100 Tours (FR); DA COSTA, Hervé, F-37170 Chambray les Tours (FR); BENNEJEAN, Caroline, F-94220 Charenton le Pont (FR); DELAGRANGE, Philippe, F-92130 Issy les Moulineaux (FR); RENARD, Pierre, F-78150 Le Chesnay (FR)
(86) Numéro de dépôt international: PCT/FR2001/002297
(87) Numéro de publication internationale: WO 2002/022555

(56) Documents cités:
- EP-A- 0 919 541
- EP-A- 0 926 145
- EP-A- 0 994 102
- WO-A-97/38682
- FR-A- 2 778 662
- US-A- 5 093 352
- US-A- 5 922 771

## Description

La présente invention concerne de nouveaux dérivés biphénylés substitués, leur procédé de préparation et les compositions pharmaceutiques qui les contiennent.

On connaît dans la littérature des composés biphénylés indoliques utiles en tant qu'inhibiteurs de métalloprotéases (WO 9615096) ou ligands spécifiques des récepteurs 5HT-1B et 5HT-1D (WO 9501334).
Des composés biphénylés benzimidazoliques sont également décrits dans la demande de brevet EP 468470 en tant qu'inhibiteurs de l'angiotensine.

Les composés de la présente invention, de par leur structure originale, sont nouveaux et présentent des propriétés pharmacologiques très intéressantes concernant les récepteurs mélatoninergiques.

On trouve dans l'art antérieur de nombreuses structures de ligands mélatoninergiques, comme par exemple les composés décrits dans WO 9738682, EP 0926145, EP 0919541, FR 2778662, EP 0994102, US 5093352 ou encore US 5922771.

De nombreuses études ont mis en évidence ces dix dernières années le rôle capital de la mélatonine (N-acétyl-5-méthoxytryptamine) dans de nombreux phénomènes physio-pathologiques ainsi que dans le contrôle du rythme circadien. Toutefois, elle possède un temps de demi-vie assez faible dû à une rapide métabolisation. Il est donc très intéressant de pouvoir mettre à la disposition du clinicien des analogues de la mélatonine, métaboliquement plus stables et présentant un caractère agoniste ou antagoniste, dont on peut attendre un effet thérapeutique supérieur à celui de l'hormone elle-même.

Outre leur action bénéfique sur les troubles du rythme circadien (J. Neurosurg. 1985, 63, pp. 321-341) et du sommeil (Psychophannacology, 1990, 100, pp. 222-226), les ligands du système mélatoninergique possèdent d'intéressantes propriétés pharmacologiques sur le système nerveux central, notamment anxiolytiques et antipsychotiques (Neuropharmacology of Pineal Secretions, 1990, 8 (3-4), pp. 264-272) et analgésiques (Pharmacopsychiat., 1987, 20, pp. 222-223) ainsi que pour le traitement des maladies de Parkinson (J. Neurosurg. 1985, 63, pp. 321-341) et d'Alzheimer (Brain Research, 1990, 528, pp. 170-174). De même, ces composés ont montré une activité sur certains cancers (Melatonin - Clinical Perspectives, Oxford University Press, 1988, pp. 164-165), sur l'ovulation (Science 1987, 227, pp. 714-720), sur le diabète (Clinical Endocrinology, 1986, 24, pp. 359-364), et dans le traitement de l'obésité (International Journal of Eating Disorders, 1996, 20 (4), pp. 443-446).
Ces différents effets s'exercent par l'intermédiaire de récepteurs spécifiques de la mélatonine. Des études de biologie moléculaire ont montré l'existence de plusieurs sous-types réceptoriels pouvant lier cette hormone (Trends Pharmacol. Sci., 1995, 16, p.50 ; WO 97.04094). Certains de ces récepteurs ont pu être localisés et caractérisés pour différentes espèces, dont les mammifères. Afin de pouvoir mieux comprendre les fonctions physiologiques de ces récepteurs, il est d'un grand intérêt de disposer de ligands spécifiques. De plus, de tels composés, en interagissant sélectivement avec l'un ou l'autre de ces récepteurs, peuvent être pour le clinicien d'excellents médicaments pour le traitement des pathologies liées au système mélatoninergique, dont certaines ont été mentionnées précédemment.

Les composés de la présente invention outre leur nouveauté, montrent une très forte affinité pour les récepteurs de la mélatonine et/ou une sélectivité pour l'un ou l'autre des sous-types réceptoriels mélatoninergiques.

Plus particulièrement, la présente invention concerne les composés de formule (I) : 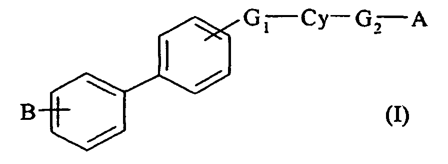 dans laquelle :
□ B représente un atome d'hydrogène ou un groupement COOR, CONRR', ou alkyle (C₁-C₆) linéaire ou ramifié substitué par un groupement COOR, CONRR' ou OR (où R et R', identiques ou différents, représentent un atome d'hydrogène, ou un groupement alkyle(C₁-C₆)linéaire ou ramifié, alkényle(C₂-C₆)linéaire ou ramifié, alkynyle (C₂-C₆)linéaire ou ramifié, aryle, arylalkyle(C₁-C₆)linéaire ou ramifié, hétéroaryle, hétéroarylalkyle(C₁-C₆)linéaire ou ramifié, polyhalogénoalkyle(C₁-C₆)linéaire ou ramifié, cycloalkyle(C₃-C₈), ou cycloalkyl(C₃-C₈)alkyle(C₁-C₆)linéaire ou ramifié, R et R' pouvant de plus former ensemble avec l'atome d'azote qui les porte un groupement morpholinyle, piperidinyle, piperazinyle ou pyrrolidinyle),
□ G₁ représente une chaîne -X'-(CH₂)ₙ-X-(CH₂)ₘ-X"- dans laquelle
   ◆ X représente un atome d'oxygène ou de soufre, ou un groupement CH₂ ou NR (dans lequel R est tel que défini précédemment),
   ◆ X' et X", identiques ou différents représentent un atome d'oxygène ou de soufre ou un groupement NR (dans lequel R est tel que défini précédemment),
   ◆ n et m, identiques ou différents, représentent 0, 1, 2, 3, 4 ou 5,
   étant entendu que l'on ne peut avoir deux hétéroatomes consécutifs et que la chaîne ainsi définie peut comporter une ou plusieurs insaturations,
□ Cy représente un groupement de formule (II) 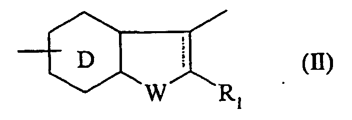 dans laquelle D représente un phényle ou une pyridine, W représente un atome d'oxygène ou de soufre, ou un groupement CH₂ ou NR (où R est tel que défini précédement), R₁ représente un atome d'halogène ou un groupement R, OR, ou COOR (où R est tel que défini précédemment) et la représentation 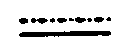 signifie que la liaison est simple ou double, étant entendu que la valence des atomes est respectée,
   ou un groupement de formule (III) 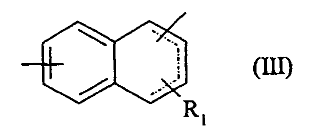 dans laquelle R₁ et la représentation 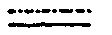 sont tels que définis précédemment,
□ G₂ représente une chaîne contenant de 1 à 6 atomes de carbone, éventuellement substituée par un ou plusieurs groupements choisis parmi R, OR, COR, COOR (où R est tel que défini précédemment), ou atomes d'halogène,
□ et A représente un groupement NRCOR', NRCSR', CONRR', CSNRR', NRCONR'R", ou NRCSNR'R" (dans lesquels R et R' sont tels que définis précédemment et R" peut prendre les mêmes valeurs que R et R'),
étant entendu que :
- par aryle on entend un groupement phényle ou naphtyle non substitué ou substitué par un ou plusieurs groupements, identiques ou différents choisis parmi R, OR, COR, COOR, NRR' (dans lesquels R et R' sont définis comme précédemment), nitro, cyano, ou atome d'halogène,
- par hétéroaryle on entend tout groupement mono ou bicyclique contenant 5 à 10 chaînons et pouvant contenir 1 à 3 hétéroatomes choisis parmi oxygène, soufre ou azote, ce groupement étant non substitué ou substitué par un ou plusieurs groupements, identiques ou différents choisis parmi R, OR, COR, COOR, NRR' (dans lesquels R et R' sont définis comme précédemment), nitro, cyano, ou atome d'halogène,
leurs énantiomères et diastéréoisomères ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, méthanesulfonique ou camphorique.

Parmi les bases pharmaceutiquement acceptables, on peut citer à titre non limitatif l'hydroxyde de sodium, l'hydroxyde de potassium, la triéthylamine ou la tertbutylamine.

Les composés préférés de l'invention sont les composés de formule (I) pour lesquels B représente :
- un groupement COOR, avec comme valeurs préférentielles pour R l'atome d'hydrogène ou le groupement alkyle(C₁-C₆) linéaire ou ramifié comme par exemple méthyle,
- ou un groupement alkyle(C₁-C₆) linéaire ou ramifié substitué par un groupement COOR ou OR, et plus préférentiellement COOH, alkyloxycarbonyle, OH ou alkoxy.

Le groupement G₁ préféré des composés de l'invention est le groupement -O-(CH₂)ₚ-O-dans lequel p est un entier tel que 0<p<6 comme par exemple -O-(CH₂)₄-O-.

Plus particulièrement, l'invention concerne les composés de formule (I) pour lesquels Cy représente un groupement naphtalène, tétrahydronaphtalène, benzothiophène, benzofurane, indole, indène, et azaindole.

G₂ représente préférentiellement un groupement -(CH₂)₂- ou -(CH₂)₃-.

Les valeurs préférées pour A sont les groupements NRCOR' et CONRR', et plus particulièrement les groupements NHCOR' et CONHR.

Encore plus préférentiellement, l'invention concerne les composés de formule (I) qui sont :
- le 4'-[4-({8-[2-(acétylamino)éthyl]-2-naphtyl}oxy)butoxy][1,1'-biphényl]-4-carboxylate de méthyle,
- l'acide 4'-[4-({8-[2-(acétylamino)éthyl]-2-naphtyl}oxy)butoxy][1,1'-biphényl]-4-carboxylique,
- le *N*-{2-[7-(4-{[4'-(hydroxyméthyl)[1,1'-biphényl]-4-yl]oxy}butoxy)-1-naphtyl]éthyl} acétamide,
- le *N*-(2-{7-[4-([1,1'-biphényl]-4-yloxy)butoxy]-1-naphtyl}éthyl)acétamide,
- le *N*-(2-{7-[4-([1,1'-biphényl]-3-yloxy)butoxy]-1-naphtyl}éthyl)acétamide.

Les énantiomères, diastéréoisomères ainsi que les sels d'addition à un acide ou une base pharmaceutiquement acceptable des composés préférés de l'invention font partie intégrante de l'invention.

La présente invention concerne également le procédé de préparation des composés de formule (I) caractérisé en ce que l'on utilise comme produit de départ le composé de formule (IV) :

MeO-Cy-G₂-A (IV)

dans laquelle A, G₂ et Cy sont tels que définis dans la formule (I),
que l'on soumet à une déméthylation en utilisant des agents classiques comme HBr, AlCl₃, AlBr₃, BBr₃ ou des systèmes binaires acide de Lewis / nucléophile comme AlCl₃ / PhCH₂SH ou BBr₃ / Me₂S par exemple, pour obtenir le composé de formule (V) :

HO-Cy-G₂-A (V)

dans laquelle A, G₂ et Cy sont définis comme précédemment,
qui est transformé, de façon classique,
- par action du *N*,*N*-diméthylthiocarbamate de sodium par exemple, en thiol correspondant de formule (VI) :

   HS-Cy-G₂-A (VI)
dans laquelle A, G₂ et Cy sont tels que définis précédemment,
- ou en dérivé aminé correspondant de formule (VII) :

   RNH-Cy-G₂-A (VII)

   dans laquelle A, G₂, Cy et R sont définis comme précédemment
les composés de formule (V), (VI) et (VII) représentant le composé de formule (VIII) :

HX"-Cy-G₂-A (VIII)

dans laquelle Cy, G₂, X" et A sont tels que définis précédemment,
composé de formule (VIII) sur lequel on condense un composé de formule (IX) : 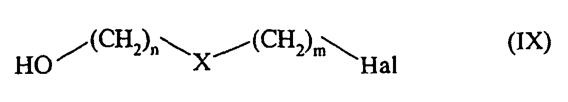 dans laquelle Hal représente un atome de brome, de chlore ou d'iode, et X, n et m sont tels que définis dans la formule (I), (étant entendu que l'on ne peut pas avoir deux hétéroatomes consécutifs et que la chaîne ainsi définie peut comporter une ou plusieurs insaturations),
pour conduire au composé de formule (X) :

HO-(CH₂)ₙ-X-(CH₂)ₘ-X"-Cy-G₂-A (X)

dans laquelle A, G₂, Cy, X, X", n et m sont définis comme précédemment (étant entendu que l'on ne peut pas avoir deux hétéroatomes consécutifs dans l'enchaînement HO-(CH₂)ₙ-X-(CH₂)ₘ-X"- et que la chaîne ainsi définie peut comporter une ou plusieurs insaturations),
dont la fonction hydroxyle est transformée classiquement en groupe partant comme par exemple un mésylate, un tosylate, ou un dérivé halogéné, pour conduire au composé de formule (X') :

E-(CH₂)ₙ-X-(CH₂)ₘ-X"-Cy-G₂-A (X')

dans laquelle A, G₂, Cy, X, X", n et m sont définis comme précédemment et E représente un groupement mésyle ou tosyle ou un atome d'halogène,
sur lequel on fait agir en milieu basique un composé de formule (XI) :

B'-Ph-Ph-X'H (XI)

dans laquelle X' est tel que défini dans la formule (1), et B' peut prendre les mêmes valeurs que B défini dans la formule (I) à l'exception des groupements COOH et alkyle substitué par un groupement COOH,
pour conduire au composé de formule (I/a), cas particulier des composés de formule (I) :

B'-Ph-Ph-G₁-Cy-G₂-A (I/a)

dans laquelle A, G₂, G₁, Cy et B' sont tels que définis précédemment,
composé de formule (I/a) étant soumis, lorsque B' représente un groupement COOR₁' ou alkyle substitué par un groupement COOR₁' (où R₁' peut prendre toutes les valeurs de R défini précédemment à l'exception de l'atome d'hydrogène), à une saponification pour conduire au composé de formule (I/b), cas particulier des composés de formule (I) :

B"-Ph-Ph-G₁-Cy-G₂-A (I/b)

dans laquelle A, G₂, G₁, et Cy sont tels que définis précédemment, et B" représente un groupement COOH ou alkyle substitué par un groupement COOH,
l'ensemble des composés (I/a) et (I/b) formant le composé de formule (I) qui peut être purifié si on le désire par une technique classique de purification, dont on sépare éventuellement les isomères selon une technique classique de séparation et que l'on transforme, le cas échéant, en leurs sels d'addition à un acide ou à une base pharmaceutiquement acceptable.

Les composés de formule (IV) sont aisément accessibles à l'homme du métier selon des méthodes décrites dans la littérature.

Les composés de l'invention et les compositions pharmaceutiques les contenant s'avèrent être utiles pour le traitement des troubles du système mélatoninergique.

L'étude pharmacologique des dérivés de l'invention a en effet montré qu'ils étaient atoxiques, doués d'une haute affinité pour les récepteurs de la mélatonine et possédaient d'importantes activités sur le système nerveux central ainsi que sur la microcirculation qui permettent d'établir que les produits de l'invention sont utiles dans le traitement du stress, des troubles du sommeil, de l'anxiété, des dépressions saisonnières, des pathologies cardiovasculaires, des pathologies du système digestif, des insomnies et fatigues dues aux décalages horaires, de la schizophrénie, des attaques de panique, de la mélancolie, des troubles de l'appétit, de l'obésité, de l'insomnie, de la douleur, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale. Dans un autre domaine d'activité, il apparaît que dans le traitement, les produits de l'invention peuvent être utilisés dans les dysfonctionnements sexuels, qu'ils possèdent des propriétés d'inhibiteurs de l'ovulation, d'immmunomodulateurs et qu'ils sont susceptibles d'être utilisés dans le traitement des cancers.

Les composés seront utilisés de préférence dans les traitements des dépressions saisonnières, des troubles du sommeil, des pathologies cardiovasculaires, des insomnies et fatigues dues aux décalages horaires, des troubles de l'appétit et de l'obésité.

Par exemple, les composés seront utilisés dans le traitement des dépressions saisonnières et des troubles du sommeil.

La présente invention a également pour objet les compositions pharmaceutiques contenant au moins un composé de formule (I) seul ou en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per. ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels denniques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,01 mg et 1 g par 24 heures en une ou plusieurs prises.

Les exemples suivants illustrent l'invention et ne la limitent en aucune façon. Les préparations suivantes conduisent à des composés de l'invention ou à des intermédiaires de synthèse utiles dans la préparation de l'invention.

### Préparation 1 : N-[2-(7-Hydroxy-1-naphtyl)éthyl]-acétamide

Sous atmosphère inerte, 27,5 mmol du complexe tribromure de bore/diméthylsulfure sont dissoutes dans 100 ml de dichlorométhane et agitées pendant 15 minutes à température ambiante. Une solution de 13,7 mmol de *N*-[2-(7-méthoxy-1-naphtyl)éthyl]acétamide dans 50 ml de dichlorométhane est ajoutée, et le milieu réactionnel est porté à reflux pendant 30 heures. Après refroidissement, la réaction est hydrolysée avec précaution et le dichlorométhane est évaporé. Le milieu est alors extrait à l'acétate d'éthyle, les phases organiques regroupées sont lavées par une solution aqueuse de bicarbonate de potassium 1M. La phase organique est séchée sur sulfate de magnésium, et concentrée pour conduire au composé du titre. Solide blanc.
Point de fusion : 125-126°C

En procédant comme dans la Préparation 1 à partir du substrat approprié, on obtient les Préparations 2 à 14 :

### Préparation 2 : N-[2-(5-Hydroxy-1-benzofuran-3-yl)éthyl]acétamide

### Préparation 3 : N-[2-(5-Hydroxy-1-benzofuran-3-yl)éthyl]cyclopropane carboxamide

### Préparation 4 : N-[2-(5-Hydroxy-1-benzofuran-3-yl)éthyl]-2-furamide

### Préparation 5 : N-[2-(7-Hydroxy-1-naphtyl)éthyl]-butanamide

### Préparation 6 : N-[2-(5-Hydroxy-1-benzothièn-3-yl)éthyl]-acétamide

### Préparation 7 : N-[2-(5-Hydroxy-1H-pyrrolo[2,3-b]pyridin-3-yl)éthyl]cyclopropane carboxamide

### Préparation 8 : N-[2-(5-Hydroxy-1H-indol-3-yl)éthyl]acétamide

### Préparation 9 : N-[2-(5-Hydroxy-1H-pyrrolo[2,3-b]pyridin-3-yl)éthyl]acétamide

### Préparation 10 : N-[2-(7-Hydroxy-1-naphtyl)éthyl]cyclobutane carboxamide

### Préparation 11 : 2,2,2-Trifluoro-N-[2-(7-hydroxy-1-naphtyl)éthyl]acétamide

### Préparation 12 : N-[2-(7-Hydroxy-1-naphtyl)éthyl]-2-furamide

### Préparation 13 : N-[2-(2-Benzyl-5-hydroxy-1H-pyrrolo[2,3-b]pyridin-3-yl)éthyl] acétamide

### Préparation 14 : N-[2-(5-Hydroxy-1H-indèn-3-yl)éthyl]pentanamide

### Préparation 15 : N-[2-(5-Mercapto-1-benzofuran-3-yl)éthyl]acétamide

A une solution d'hydroxyde de potassium (10 mmol) dissoute dans 15 ml d'eau et 16 ml de tétrahydrofurane, on additionne le produit obtenu dans la Préparation 2 (9 mmol) en maintenant l'agitation. La solution est refroidie à l'aide d'un bain de glace et de sel et on ajoute goutte à goutte le chlorure de diméthylthiocarbamoyle (9 mmol) en solution dans le tétrahydrofurane (15 ml) sous agitation. Après une demi-heure d'agitation en maintenant le froid, le milieu réactionnel est extrait au chloroforme. Les phases organiques sont regroupées, séchées sur sulfate de magnésium, filtrées puis concentrées sous pression réduite. Le résidu est repris dans le diphényléther (10 ml) et porté au reflux pendant une heure sous atmosphère d'azote. Le diphényléther est évaporé sous pression réduite jusqu'à obtention d'une solution d'environ 2 ml. Les 2 ml de distillat encore chauds sont versés avec précaution dans 50 ml d'hexane pour donner après refroidissement un solide isolé par filtration. Le solide ainsi collecté est additionné à une solution d'hydroxyde de potassium (380 mg) dissous dans un mélange eau/méthanol (1 ml/10 ml). La solution est portée au reflux pendant 12 heures puis refroidie et concentrée sous pression réduite. Le résidu est repris avec 20 ml de chloroforme et extrait 3 fois à l'eau. La phase organique est séchée sur sulfate de magnésium, filtrée et concentrée sous pression réduite. Le résidu est chromatographié sur gel de silice pour conduire au produit du titre.

### Préparation 16 : N-[2-(5-Amino-1-benzofuran-3-yl)éthyl]acétamide

### Stade A : N-[2-(5-Bromo-1-benzofuran-3-yl)éthyl]acétamide

Dans un tricol de 150 ml équipé d'une ampoule à brome, d'un réfrigérant surmonté d'un tube rempli de chlorure de calcium, et d'un agitateur mécanique, on verse de la triphénylphosphine (10 mmol) et de l'acétonitrile (70 ml). La solution est refroidie à l'aide d'un bain de glace en maintenant l'agitation et on additionne le brome (10 mmol). A la fin de l'addition, le bain de glace est retiré puis on ajoute le produit obtenu dans la Préparation 2 (8 mmol). Le mélange réactionnel est agité à 60-70°C jusqu'à disparition du produit de départ. En fin de réaction, le mélange est filtré puis le filtrat est concentré sous pression réduite. Le résidu est repris dans l'acétate d'éthyle, lavé à l'eau puis avec une solution saturée d'hydrogénocarbonate de potassium, et encore une fois à l'eau puis séché sur sulfate de magnésium et concentré sous pression réduite. Le résidu est filtré sur gel de silice pour conduire au produit du titre.

### Stade B : N-[2-(5-Iodo-1-benzofuran-3-yl)éthyl]acétamide

Un mélange du produit obtenu au stade A (2 mmol), d'iodure de potassium (30 mmol) et d'iodure de cuivre 1 (10 mmol) dans l'hexaméthyl phosphoramide (6 ml) est chauffé à 150-160°C avec agitation sous atmosphère d'azote jusqu'à ce qu'un taux de conversion de 90 % soit atteint. On ajoute alors de l'acide chlorhydrique dilué puis de l'éther et la mixture est alors filtrée pour éliminer les sels de cuivre I insolubles. La phase organique est séparée, lavée avec une solution de sulfite de sodium, de l'eau, séchée sur sulfate de magnésium et évaporée pour donner un résidu que l'on chromatographie sur gel de silice pour conduire au produit du titre.

### Stade C : N-[2-(5-Vinyl-1-benzofuran-3-yl)éthyl]acétamide

15 mmol du produit obtenu au stade B, 16 mmol de vinyl tributylétain et 0,43 mmol de (triphénylphosphine) palladium tetrakis, sont portés sous agitation à 110°C pendant 3 heures dans 30 ml de *N*-méthylpyrrolidinone. Après évaporation du solvant, le résidu est repris dans 20 ml de dichlorométhane et traité par une solution aqueuse à 10 % de fluorure de potassium. Après extraction, concentration sous pression réduite et chromatographie sur gel de silice, on obtient le produit du titre pur.

### Stade D : N-[2-(5-Formyl-1-benzofuran-3-yl)éthyl]acétamide

A une solution de 10 mmol du produit obtenu dans le stade C dans un mélange de 50 ml de dioxane et 25 ml d'eau sont ajoutés à température ambiante 1,10 g de tétroxyde d'osmium dans le 2-méthyl-2-propanol, puis 8,70 g de Periodate de sodium. Après agitation une nuit à température ambiante, la suspension est filtrée, le filtrat concentré sous pression réduite. Le résidu obtenu est repris dans le dichlorométhane. La phase organique est lavée avec de l'eau, séchée et évaporée. Le résidu est purifié par chromatographie sur gel de silice pour conduire au produit du titre.

### Stade E : Acide 3-[2-(acétylamino)éthyl]-1-benzofuran-5-carboxylique

A une solution de 6,88 mmol du produit obtenu dans le stade D dans 30 ml d'acétone sont ajoutés à température ambiante 2,7 g de permanganate de potassium dans 50 ml d'un mélange acétone/eau (50/50). La solution est agitée 2 heures à température ambiante puis filtrée. Le filtrat est concentré sous pression réduite et chromatographié sur gel de silice pour conduire au produit du titre.

### Stade F : Chlorure de l'acide 3-[2-(acétylamino)éthyl]-1-benzofuran-5-carboxylique

5 mmol du produit obtenu dans le stade E sont dissoutes dans 40 ml de chlorure de thionyle. Après agitation sous atmosphère inerte pendant 1 heure, le chlorure de thionyle est évaporé sous pression réduite pour conduire au produit du titre.

### Stade G : N-[2-(5-Amino-1-benzofuran-3-yl)éthyl]acétamide

Une solution du produit obtenu dans le stade F (20 mmol) dans le dichlorométhane (30 ml) contenant du bromure de tétrabutyl ammonium (20 mg) est refroidie dans un bain de glace. Après addition de l'azoture de sodium (25 mmol) dissous dans 5 ml d'eau la solution est agitée vigoureusement à 0°C pendant 2 heures. La phase organique est séparée, lavée à l'eau (2 x 5 ml) et séchée sur sulfate de magnésium. Après filtration, on ajoute l'acide trifluoroacétique (30 mmol) et la solution est agitée sous reflux pendant 60 heures. Après refroidissement, la phase organique est lavée avec une solution saturée d'hydrogénocarbonate de sodium (2 x 5 ml) et concentrée sous pression réduite. Le résidu est alors repris dans le méthanol (20 ml) et on ajoute de l'eau (80 ml) puis du carbonate de potassium (30 mmol). Après agitation à température ambiante pendant 20 heures, le milieu réactionnel est concentré sous pression réduite jusqu'à un volume de 60 ml environ puis extrait 3 fois à l'éther (3 x 50 ml). Après séchage sur sulfate de sodium, la phase organique est filtrée puis évaporée sous pression réduite. Le résidu est chromatographié sur gel de silice pour conduire au produit du titre.

### Préparation 17 : N-[2-(5-Amino-1-benzofuran-3-yl)éthyl]-2-furamide

On procède comme dans la Préparation 16 à partir du composé obtenu dans la Préparation 4.

### Préparation 18 : N-[2-(5-Hydroxy-1-méthyl-1H-pyrrolo[2,3-b]pyridin-3-yl)éthyl] acétamide

On procède comme dans la Préparation 1.

### Préparation 19 : N-[2-(7-Hydroxy-1,2,3,4-tétrahydro-1-naphtalényl)éthyl]acétamide

On procède comme dans la Préparation 1.

### Préparation 20 : N-[2-(5-Hydroxy-1-méthyl-1H-pyrrolo[3,2-b]pyridin-3-yl)éthyl] acétamide

On procède comme dans la Préparation 1.

### Exemple 1 : 4'-[2-({8-[2-(Acétylamino)éthyl]-2-naphtyl}oxy)éthoxy][1,1'-biphényl]-4-carboxylate de méthyle

### Stade A : N-{2-[7-(2-Bromoéthoxy)napht-1-yl]éthyl}acétamide

On dissout le composé obtenu dans la Préparation 1 (0,009 mol) dans 20 ml d'un mélange de diméthylsulfoxyde (6 ml) et de butanone (14 ml). On ajoute 0,027 mol de carbonate de potassium et 0,036 mol de dibromoéthane, puis on chauffe à reflux pendant 48 heures. Le milieu réactionnel est ensuite refroidi et versé dans l'eau. La phase aqueuse est extraite par Et₂O, puis la phase organique est lavée à l'eau jusqu'à neutralité des eaux de lavage, puis séchée sur sulfate de magnésium et évaporée sous pression réduite. Le résidu obtenu est purifié par chromatographie sur gel de silice (éluant : acétone/cyclohexane (2/8)) et recristallisé. Solide blanc.
Point de fusion: 110-111°C

| Microanalyse élémentaire | | | |
|---|---|---|---|
| % | C | H | N |
| Calculé | 57,15 | 5,40 | 4,17 |
| Trouvé | 57,28 | 5,38 | 3,91 |

### Stade B : 4'-[2-({8-[2-(Acétylamino)éthyl]-2-naphtyl}oxy)éthoxy][1,1'-biphényl]-4-carboxylate de méthyle

Dans un ballon de 100 ml, on dissout 0,003 mol de 4'-hydroxy[1,1'-biphenyl]-4-carboxylate de méthyle et 0,003 mol du composé obtenu au stade A dans un mélange constitué de 3 ml de diméthylsulfoxyde et 20 ml de butanone. On ajoute 0,009 mol de carbonate de potassium et un cristal d'iodure de potassium puis on chauffe à reflux pendant 12 heures. Le milieu réactionnel est ensuite refroidi et versé dans 100 ml d'eau. Le précipité formé est essoré et recristallisé pour conduire au produit du titre.

### Exemple 2 : Acide 4'-[2-({8-[2-(acétylamino)éthyl]-2-naphtyl}oxy)éthoxy][1,1'-biphényl]-4-carboxylique

Dans un ballon de 100 ml, on met en suspension 19 mmol du composé obtenu dans l'Exemple 1 dans 25 ml de THF, puis on ajoute 15 ml de méthanol, 15 ml d'eau et 38 mmol de soude. Le milieu réactionnel est maintenu sous agitation à température ambiante pendant 10 heures. La solution est ensuite concentrée, versée dans l'eau, puis acidifiée avec HCl 12M. Le précipité obtenu est filtré, lavé à l'eau et recristallisé pour conduire au produit du titre

### Exemple 3 : 4'-[2-({3-[2-(Acétylamino)ethyl]-1-benzofuran-5-yl}oxy)éthoxy][1,1'-biphényl]-4-carboxylate de méthyle

On procède comme dans l'Exemple 1 en remplaçant dans le stade A le composé obtenu dans la Préparation 1 par le composé obtenu dans la Préparation 2.

### Exemple 4 : 4'-{2-[(3-{2-[(Cyclopropylcarbonyl)amino]éthyl}-1-benzofuran-5-yl)oxy]éthoxy}[1,1'-biphényl]-4-carboxylate de méthyle

On procède comme dans l'Exemple 1 en remplaçant au stade A le composé obtenu dans la Préparation 1 par le composé obtenu dans la Préparation 3.

### Exemple 5 : 4'-[2-({3-[2-(2-Furoylamino)éthyl]-1-benzofuran-5-yl}oxy)éthoxy][1,1'-biphényl]-4-carboxylate de méthyle

On procède comme dans l'Exemple 1 en remplaçant au stade A le composé obtenu dans la Préparation 1 par le composé obtenu dans la Préparation 4.

### Exemple 6 : 4'-[2-({3-[2-(Acétylamino)éthyl]-1-benzofuran-5-yl}amino)éthoxy][1,1'-biphényl]-4-carboxylate de méthyle

On procède comme dans l'Exemple 1 en remplaçant dans le stade A le composé obtenu dans la Préparation 1 par le composé obtenu dans la Préparation 16.

### Exemple 7 : 4'-[2-({8-[2-(Butyrylamino)éthyl]-2-naphtyl}oxy)éthoxy][1,1'-biphényl]-4-carboxylate de méthyle

On procède comme dans l'Exemple 1 en remplaçant dans le stade A le composé obtenu dans la Préparation 1 par le composé obtenu dans la Préparation 5.

### Exemple 8 : 4'-[2-({3-[2-(Acétylamino)éthyl]-1-benzothièn-5-yl}oxy)éthoxy][1,1'-biphényl]-4-carboxylate de méthyle

On procède comme dans l'Exemple 1 en remplaçant dans le stade A le composé obtenu dans la Préparation 1 par le composé obtenu dans la Préparation 6.

### Exemple 9 : 4'-{2-[(3-{2-[(Cyclopropylcarbonyl)amino]éthyl}-1H-pyrrolo[2,3-b]pyridin-5-yl)oxy]éthoxy}[1,1'-biphényl]-4-carboxylate de méthyle

On procède comme dans l'Exemple 1 en remplaçant dans le stade A le produit obtenu dans la Préparation 1 par le composé obtenu dans la Préparation 7.

### Exemple 10 : N-{2-[7-(2-{[4'-(Hydroxyméthyl)[1,1'-biphényl]-4-yl]oxy}éthoxy)-1-naphtyl]éthyl}acétamide

On procède comme dans l'Exemple 1 en remplaçant dans le stade B le 4'-hydroxy[1,1'-biphenyl]-4-carboxylate de méthyle par le 4'-(hydroxyméthyl)[1,1'-biphényl]-4-ol.

### Exemple 11 : 4'-[2-({3-[2-(Acétylamino)éthyl]-1H-indol-5-yl}oxy)éthoxy][1,1'-biphényl]-4-carboxylate de méthyle

On procède comme dans l'Exemple 1 en remplaçant dans le stade A, le composé obtenu dans la Préparation 1 par le composé obtenu dans la Préparation 8.

### Exemple 12 : Acide 4'-[2-({3-[2-(acétylamino)éthyl]-1H-indol-5-yl}oxy)éthoxy][1,1'-biphényl]-4-carboxylique

On procède comme dans l'Exemple 2 à partir du composé obtenu dans l'Exemple 11.

### Exemple 13 : 4'-[3-({8-(2-(Acétylamino)éthyl]-2-naphtyl}oxy)propoxy][1,1'-biphényl]-4-carboxylate de méthyle

### Stade A : N-{2-[7-(3-Hydroxypropyloxy)napht-1-yl]éthyl}acétamide

Dans un ballon de 100 ml, on dissout 0,022 mol du composé obtenu dans la Préparation 1 dans 30 ml de diméthylformamide. On ajoute 0,066 mol de carbonate de potassium et 0,033 mol de 3-bromopropan-1-ol, puis le mélange est chauffé à 80°C pendant 4 heures. Le milieu réactionnel est refroidi et versé dans 100 ml d'une solution d'HCl 1M. La phase aqueuse est extraite 3 fois avec Et₂O puis la phase organique est séchée sur MgSO₄ et évaporée sous pression réduite. Le produit du titre est obtenu après recristallisation. Solide blanc.
Point de fusion : 141-142°C

### Stade B : 3-({8-[2-(Acétylamino)éthyl]-2-naphtyl}oxy)propylméthanesulfonate

Dans un ballon de 250 ml, l'alcool obtenu au stade A est dissous dans 50 ml de dichlorométhane et on ajoute 0,012 mol de triéthylamine. On refroidit dans un bain de glace-sel à -10°C puis 0,012 mol de chlorure de mésyle sont ajoutées goutte à goutte sous agitation magnétique. Le milieu réactionnel est agité à température ambiante pendant 4 heures. Puis on additionne 100 ml d'eau et on poursuit par une extraction au CH₂Cl₂. La phase organique est lavée à l'eau, séchée sur MgSO₄ et évaporée sous pression réduite. L'huile obtenue est purifiée par chromatographie sur gel de silice (éluant : acétone/cyclohexane (2/8)).

### Stade C : 4'-[3-({8-[2-(Acétylamino)éthyl]-2-naphtyl}oxy)propoxy][1,1'-biphényl]-4-carboxylate de méthyle

Dans un ballon de 100 ml contenant 30 ml de méthanol, on ajoute par petites portions 0,06 g de sodium. Lorsque le sodium est totalement consommé, on ajoute 0,0033 mol de 4'-hydroxy[1,1'-biphenyl]-4-carboxylate de méthyle et on laisse agiter pendant 20 minutes. Le méthanol est évaporé sous pression réduite et le résidu est repris dans 15 ml de DMF, puis on ajoute 0,0027 mol du composé obtenu au stade B. Le milieu réactionnel est ensuite chauffé à reflux pendant 12 heures puis refroidi et versé dans 100 ml d'eau et 10 ml d'HCl 3M. Après extraction à l'acétate d'éthyle, la phase organique est lavée avec une solution de soude 10 % puis à l'eau. Après séchage sur MgSO₄, évaporation sous pression réduite du solvant, le composé du titre est purifié par chromatographie sur gel de silice.

### Exemple 14: 4'-{3-[(8-{2-[(Cyclobutylcarbonyl)amino]éthyl}-2-naphtyl)oxy] propoxy}[1,1'-biphényl]-4-carboxylate de méthyle

On procède comme dans l'Exemple 13 à partir du composé obtenu dans la Préparation 10.

### Exemple 15: 4'-[3-({3-[2-(2-Furoylamino)éthyl]-1-benzofuran-5-yl}amino) propoxy][1,1'-biphényl]-4-carboxylate de méthyle

On procède comme dans l'Exemple 13 à partir du composé obtenu dans la Préparation 17.

### Exemple 16 : Acide 4'-[3-({3-[2-(2-furoylamino)éthyl]-1-benzofuran-5-yl}amino propoxy][1,1'-biphényl]-4-carboxylique

On procède comme dans l'Exemple 2 à partir du composé obtenu dans l'Exemple 15.

### Exemple 17 : 4'-{3-[(8-{2-[(Trifluoroacétyl)amino]éthyl}-2-naphtyl)oxy]propoxy} [1,1'-biphényl]-4-carboxylate de méthyle

On procède comme dans l'Exemple 13 en remplaçant le composé de la Préparation 1 par le composé de la Préparation 11.

### Exemple 18 : Acide 4'-{3-[(8-{2-[(trifluoroacétyl)amino]éthyl}-2-naphtyl)oxy] propoxy}[1,1'-biphényl]-4-carboxylique

On procède comme dans l'Exemple 2 à partir du composé obtenu dans l'Exemple 17.

### Exemple 19 : 4'-[4-({8-[2-(Acétylamino)éthyl]-2-naphtyl}oxy)butoxy][1,1'-biphényl]-4-carboxylate de méthyle

### Stade A : N-{2-[7-(4-Bromobutoxy)-1-naphtyl]éthyl}acétamide

On procède comme dans le stade A de l'Exemple 1 en remplaçant le dibromoéthane par le 1,4-dibromobutane.

### Stade B : 4'-[4-({8-[2-(Acétylamino)éthyl]-2-naphtyl}oxy)butoxy][1,1'-biphényl]-4-carboxylate de méthyle

On procède comme dans le stade B de l'Exemple 1.
Solide blanc.
Point de fusion : 166-168°C

| Microanalyse élémentaire : | | | |
|---|---|---|---|
| % | C | H | N |
| Calculé | 74,61 | 6,45 | 2,72 |
| Trouvé | 74,62 | 6,48 | 2,81 |

### Exemple 20 : Acide 4'-[4-({8-[2-(acétylamino)éthyl]-2-naphtyl}oxy)butoxy][1,1'-biphényl]-4-carboxylique

On procède comme dans l'Exemple 2 à partir du composé obtenu dans l'Exemple 19.
Solide blanc.
Point de fusion : 223-225°C

| Microanalyse élémentaire : | | | |
|---|---|---|---|
| % | C | H | N |
| Calculé | 70,83 | 5,94 | 2,66 |
| Trouvé | 71,16 | 6,05 | 2,69 |

### Exemple 21 : N-{2-[7-(4-{[4'-(Hydroxyméthyl)[1,1'-biphényl]-4-yl]oxy}butoxy)-1-naphtyl]éthyl}acétamide

On procède comme dans l'Exemple 19 en remplaçant dans le stade B le 4'-hydroxy[1,1'-biphenyl]-4-carboxylate de méthyle par le 4'-(hydroxyméthyl)[1,1'-biphényl]-4-ol.
Solide beige.
Point de fusion: 172-173°C

| Microanalyse élémentaire : | | | |
|---|---|---|---|
| % | C | H | N |
| Calculé | 76,99 | 6,88 | 2,90 |
| Trouvé | 76,74 | 6,70 | 3,12 |

### Exemple 22 : N-(2-{7-[4-([1,1'-Biphényl]-4-yloxy)butoxy]-1-naphtyl}éthyl)acétamide

On procède comme dans l'Exemple 19 en remplaçant dans le stade B le 4'-hydroxy[1,1'-biphenyl]-4-carboxylate de méthyle par le (1,1'-biphényl)-4-ol.
Solide blanc.
Point de fusion : 138-140°C

| Microanalyse élémentaire : | | | |
|---|---|---|---|
| % | C | H | N |
| Calculé | 79,44 | 6,89 | 3,10 |
| Trouvé | 79,19 | 6,93 | 3,24 |

### Exemple 23 : N-(2-{7-[4-([1,1'-Biphényl]-3-yloxy)butoxy]-1-naphtyl}éthyl)acétamide

On procède comme dans l'Exemple 19 en remplaçant dans le stade B le 4'-hydroxy[1,1'-biphenyl]-4-carboxylate de méthyle par le (1,1'-biphényl)-3-ol.
Solide blanc.
Point de fusion : 111-112°C

| Microanalyse élémentaire : | | | |
|---|---|---|---|
| % | C | H | N |
| Calculé | 79,44 | 6,89 | 3,10 |
| Trouvé | 79,23 | 6,79 | 3,21 |

### Exemple 24 : 4'-[4-({3-[2-(Acétylamino)éthyl]-1-benzofuran-5-yl}oxy)butoxy][1,1'-biphényl]-4-carboxylate de méthyle

On procède comme dans l'Exemple 19 à partir du composé obtenu dans la Préparation 2.

### Exemple 25 : 4'-[4-({3-[2-(Acétylamino)éthyl]-1-benzothièn-5-yl}oxy)butoxy][1,1'-biphényl]-4-carboxylate de méthyle

On procède comme dans l'Exemple 19 à partir du composé obtenu dans la Préparation 6.

### Exemple 26 : 4'-[4-({3-[2-(Acétylamino)éthyl]-1H-pyrrolo[2,3-b]pyridin-5-yl} oxy)butoxy][1,1'-biphényl]-4-carboxylate de méthyle

On procède comme dans l'Exemple 19 à partir du composé obtenu dans la Préparation 9.

### Exemple 27 : Acide 4'-[4-({3-[2-(acétylamino)éthyl]-1-benzofuran-5-yl}oxy)butoxy] [1,1'-biphényl]-4-carboxylique

On procède comme dans l'Exemple 2 à partir du composé obtenu dans l'Exemple 24.

### Exemple 28 : Acide 4'-[4-({3-[2-(acétylamino)éthyl]-1-benzothièn-5-yl}oxy)butoxy [1,1'-biphényl]-4-carboxylique

On procède comme dans l'Exemple 2 à partir du composé obtenu dans l'Exemple 25.

### Exemple 29 : Acide 4'-[4-({3-[2-(acétylamino)éthyl]-1H-pyrrolo[2,3-b]pyridin-5-yl}oxy)butoxy][1,1'-biphényl]-4-carboxylique

On procède comme dans l'Exemple 2 à partir du composé obtenu dans l'Exemple 26.

### Exemple 30 : 4'-[4-({3-[2-(Acétylamino)éthyl]-2-benzyl-1H-pyrrolo[2,3-b]pyridin-5-yl}oxy)butoxy][1,1'-biphényl]-4-carboxylate de méthyle

On procède comme dans l'Exemple 19 à partir du composé obtenu dans la Préparation 13.

### Exemple 31 : 4'-[4-({8-[2-(2-Furoylamino)éthyl]-2-naphtyl}oxy)butoxy][1,1'-biphényl]-4-carboxylate de méthyle

On procède comme dans l'Exemple 19 à partir du composé obtenu dans la Préparation 12.

### Exemple 32 : 4'-[4-({3-[2-(Pentanoylamino)éthyl]-1H-indèn-5-yl}oxy)butoxy][1,1'-biphényl]-4-carboxylate de méthyle

On procède comme dans l'Exemple 19 à partir du composé obtenu dans la Préparation 14.

### Exemple 33 : 4'-[4-({3-[2-(Acétylamino)éthyl]-1-benzofuran-5-yl}thio)butoxy][1,1'-biphényl]-4-carboxylate de méthyle

On procède comme dans l'Exemple 19 à partir du composé obtenu dans la Préparation 15.

### Exemple 34 : 4'-[4-({3-[2-(Acétylamino)éthyl]-1-méthyl-1H-pyrrolo[2,3-b]pyridin-5-yl}oxy)butoxy][1,1'-biphényl]-4-carboxylate de méthyle

On procède comme dans l'Exemple 19 à partir du composé obtenu dans la Préparation 18.

### Exemple 35: 3'-[4-({8-[2-(Acétylamino)éthyl]-5,6,7,8-tétrahydro-2-naphtalényl} oxy) butoxy][1,1'-biphenyl]-3-carboxylate de méthyle

On procède comme dans l'Exemple 19 à partir du composé obtenu dans la Préparation 19.

### Exemple 36 : Acide 3'-[4-({8-[2-(acétylamino)éthyl]-5,6,7,8-tétrahydro-2-naphtalényl}oxy)butoxy][1,1'-biphényl]-3-carboxylique

On procède comme dans l'Exemple 2 à partir du composé obtenu dans l'Exemple 35.

### Exemple 37 : 4'-[4-({3-[2-(Acétylamino)éthyl]-1-méthyl-1H-pyrrolo[3,2-b]pyridin-5-yl}oxy)butoxy][1,1'-biphényl]-4-carboxylate d'éthyle

On procède comme dans l'Exemple 19 à partir du composé obtenu dans la Préparation 20 et de 4'-hydroxy[1,1'-biphényl]-4-carboxylate d'éthyle.
Point de fusion: 145-146°C

| Microanalyse élémentaire : | | | |
|---|---|---|---|
| % | C | H | N |
| Calculé | 70,30 | 6,66 | 7,93 |
| Trouvé | 69,84 | 6,67 | 7,94 |

### ETUDE PHARMACOLOGIQUE

### EXEMPLE A : Etude de la toxicité aiguë

La toxicité aiguë a été appréciée après administration orale à des lots de 8 souris (26 ± 2 grammes). Les animaux ont été observés à intervalles réguliers au cours de la première journée et quotidiennement pendant les deux semaines suivant le traitement. La DL 50, entraînant la mort de 50 % des animaux, a été évaluée et a montré la faible toxicité des composés de l'invention.

### EXEMPLE B : Etude de liaison aux récepteurs de la mélatonine sur des cellules de la Pars tuberalis de mouton

Les études de liaison aux récepteurs de la mélatonine des composés de l'invention ont été réalisées selon les techniques classiques sur les cellules de la *Pars tuberalis* de mouton. La *Pars tuberalis* de l'adénohypophyse est en effet caractérisée, chez les mammifères, par une haute densité en récepteurs de la mélatonine (Journal of Neuroendocrinology, 1, pp. 1-4, 1989).

### Protocole

1) Les membranes de *Pars tuberalis* de mouton sont préparées et utilisées comme tissu cible dans des expériences de saturation pour déterminer les capacités et affinités de liaison pour la 2-[¹²⁵I]-iodomélatonine.
2) Les membranes de *Pars tuberalis* de mouton sont utilisées comme tissu cible, avec les différents composés à tester, dans des expériences de liaison compétitive par rapport à la mélatonine.

Chaque expérience est réalisée en triple et une gamme de concentrations différentes est testée pour chaque composé. Les résultats permettent de déterminer, après traitement statistique, les affinités de liaison du composé testé.

### Résultats

Il apparaît que les composés de l'invention possèdent une puissante affinité pour les récepteurs de la mélatonine.

### EXEMPLE C : Etude de liaison aux récepteurs MT₁ et MT₂ de la mélatonine

Les expériences de liaison aux récepteurs MT₁ ou MT₂ sont réalisées en utilisant la 2-[¹²⁵I]-iodomélatonine comme radioligand de référence. La radioactivité retenue est déterminée à l'aide d'un compteur à scintillation liquide.

Des expériences de liaison compétitive sont ensuite réalisées en triple, avec les différents composés à tester. Une gamme de concentrations différentes est testée pour chaque composé. Les résultats permettent de déterminer les affinités de liaison des composés testés (IC₅₀).

Ainsi, les valeurs d'IC₅₀ trouvées pour les composés de l'invention attestent d'une liaison pour l'un ou l'autre des sous-types réceptoriels MT₁ ou MT₂, ces valeurs étant ≤ 10 µM.

### EXEMPLE D : Action des composés de l'invention sur les rythmes circadiens d'activité locomotrice du rat

L'implication de la mélatonine dans l'entraînement, par l'alternance jour/nuit, de la plupart des rythmes circadiens physiologiques, biochimiques et comportementaux a permis d'établir un modèle pharmacologique pour la recherche de ligands mélatoninergiques.

Les effets des molécules sont testés sur de nombreux paramètres et, en particulier, sur les rythmes circadiens d'activité locomotrice qui représentent un marqueur fiable de l'activité de l'horloge circadienne endogène.

Dans cette étude, on évalue les effets de telles molécules sur un modèle expérimental particulier, à savoir le rat placé en isolement temporel (obscurité permanente).

### Protocole expérimental

Des rats mâles âgés de un mois sont soumis dès leur arrivée au laboratoire à un cycle lumineux de 12h de lumière par 24h (LD 12 : 12).
Après 2 à 3 semaines d'adaptation, ils sont placés dans des cages équipées d'une roue reliée à un système d'enregistrement afin de détecter les phases d'activité locomotrice et de suivre ainsi les rythmes nycthéméraux (LD) ou circadiens (DD).

Dès que les rythmes enregistrés témoignent d'un entraînement stable par le cycle lumineux LD 12 : 12, les rats sont mis en obscurité permanente (DD).

Deux à trois semaines plus tard, lorsque le libre-cours (rythme reflétant celui de l'horloge endogène) est clairement établi, les rats reçoivent une administration quotidienne de la molécule à tester.

Les observations sont réalisées grâce à la visualisation des rythmes d'activité :
- entraînement des rythmes d'activité par le rythme lumineux,
- disparition de l'entraînement des rythmes en obscurité permanente,
- entraînement par l'administration quotidienne de la molécule ; effet transitoire ou durable.

Un logiciel permet :
- de mesurer la durée et l'intensité de l'activité, la période du rythme chez les animaux en libre cours et pendant le traitement,
- de mettre éventuellement en évidence par analyse spectrale l'existence de composants circadiens et non circadiens (ultradiens par exemple).

### Résultats

Il apparaît clairement que les composés de l'invention permettent d'agir de façon puissante sur le rythme circadien *via* le système mélatoninergique.

### EXEMPLE E : Test des cages claires/obscures

Les composés de l'invention sont testés dans un modèle comportemental, le test des cages claires/obscures, qui permet de révéler l'activité anxiolytique des molécules.

L'appareil est composé de deux boîtes en polyvinyle couvertes de Plexiglas. L'une de ces boîtes est obscure. Une lampe est placée au-dessus de l'autre boîte donnant une intensité lumineuse au centre de celle-ci d'approximativement 4000 lux. Un tunnel opaque en plastique sépare la boîte claire de la boîte sombre. Les animaux sont testés individuellement pendant une session de 5 min. Le plancher de chaque boîte est nettoyé entre chaque session. Au début de chaque test, la souris est placée dans le tunnel, face à la boîte sombre. Le temps passé par la souris dans la boîte éclairée et le nombre de transitions à travers le tunnel sont enregistrés après la première entrée dans la boîte sombre.

Après administration des composés 30 min avant le début du test, les composés de l'invention augmentent de façon significative le temps passé dans la cage éclairée ainsi que le nombre des transitions, ce qui montre l'activité anxiolytique des dérivés de l'invention.

### EXEMPLE F : Activité des composés de l'invention sur l'artère caudale de rat

Les composés de l'invention ont été testés *in vitro* sur l'artère caudale de rat. Les récepteurs mélatoninergiques sont présents sur ces vaisseaux ce qui en fait un modèle pharmacologique relevant pour étudier l'activité de ligands mélatoninergiques. La stimulation des récepteurs peut induire soit une vasoconstriction soit une dilatation en fonction du segment artériel étudié.

### Protocole

Des rats âgés de 1 mois sont habitués pendant 2 à 3 semaines à un cycle lumière/obscurité 12h/12h.
Après sacrifice, l'artère caudale est isolée et maintenue dans un milieu richement oxygéné. Les artères sont ensuite cannulées aux deux extrémités, suspendues verticalement dans une chambre d'organe dans un milieu approprié et perfusées via leur extrémité proximale. Les changements de pression dans le débit de la perfusion permettent d'évaluer l'effet vasoconstricteur ou vasodilatateur des composés.
L'activité des composés est évaluée sur des segments pré-contractés par la phényléphrine (1 µM). Une courbe concentration-réponse est déterminée de façon non-cumulative par addition d'une concentration du composé étudié sur le segment pré-contracté. Lorsque l'effet observé a atteint l'équilibre, le milieu est changé et la préparation laissée 20 minutes avant l'addition d'une même concentration de phényléphrine et d'une nouvelle concentration du composé étudié.

### Résultats

Les composés de l'invention modifient de façon significative le diamètre des artères caudales préconstrictées par la phényléphrine.

### EXEMPLE G : Composition pharmaceutique : Comprimés

| | |
|---|---|
| 1000 comprimés dosés à 5 mg d'acide 4-[4-({8-[2-(acétylamino)éthyl]-2-naphtyl} oxy)butoxy][1,1'-biphényl]-4-carboxylique (Exemple 20) | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I) : 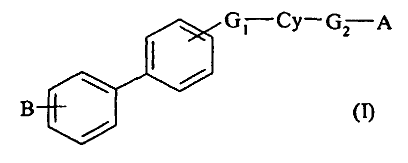 dans laquelle :
□ B représente un atome d'hydrogène ou un groupement COOR, CONRR', ou alkyle (C₁-C₆) linéaire ou ramifié substitué par un groupement COOR, CONRR' ou OR (où R et R', identiques ou différents, représentent un atome d'hydrogène, ou un groupement alkyle(C₁-C₆)linéaire ou ramifié, alkényle(C₂-C₆)linéaire ou ramifié, alkynyle (C₂-C₆)linéaire ou ramifié, aryle, arylalkyle(C₁-C₆)linéaire ou ramifié, hétéroaryle, hétéroarylalkyle(C₁-C₆)linéaire ou ramifié, polyhalogénoalkyle(C₁-C₆)linéaire ou ramifié, cycloalkyle(C₃-C₈), ou cycloalkyl(C₃-C₈)alkyle(C₁-C₆)linéaire ou ramifié, R et R' pouvant de plus former ensemble avec l'atome d'azote qui les porte un groupement morpholinylé, piperidinyle, piperazinyle ou pyrrolidinyle),
□ G₁ représente une chaîne -X'-(CH₂)ₙ-X-(CH₂)ₘ-X"- dans laquelle
◆ X représente un atome d'oxygène ou de soufre, ou un groupement CH₂ ou NR (dans lequel R est tel que défini précédemment),
◆ X' et X", identiques ou différents représentent un atome d'oxygène ou de soufre ou un groupement NR (dans lequel R est tel que défini précédemment),
◆ n et m, identiques ou différents, représentent 0, 1, 2, 3, 4 ou 5,
étant entendu que l'on ne peut avoir deux hétéroatomes consécutifs et que la chaîne ainsi définie peut comporter une ou plusieurs insaturations,
□ Cy représente un groupement de formule (II) 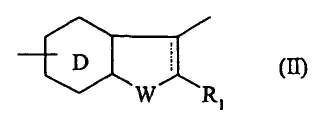 dans laquelle D représente un phényle ou une pyridine, W représente un atome d'oxygène ou de soufre, ou un groupement CH₂ ou NR (où R est tel que défini précédement), R₁ représente un atome d'halogène ou un groupement R, OR, ou COOR (où R est tel que défini précédemment) et la représentation 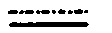 signifie que la liaison est simple ou double, étant entendu que la valence des atomes est respectée,
ou un groupement de formule (III) 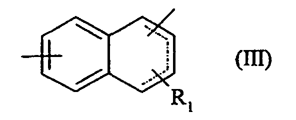 dans laquelle R₁ et la représentation 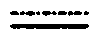 sont tels que définis précédemment,
□ G₂ représente une chaîne contenant de 1 à 6 atomes de carbone, éventuellement substituée par un ou plusieurs groupements choisis parmi R, OR, COR, COOR (où R est tel que défini précédemment), ou atomes d'halogène,
□ et A représente un groupement NRCOR', NRCSR', CONRR', CSNRR', NRCONR'R", ou NRCSNR'R" (dans lesquels R et R' sont tels que définis précédemment et R" peut prendre les mêmes valeurs que R et R'),
étant entendu que :
• par aryle on entend un groupement phényle ou naphtyle non substitué ou substitué par un ou plusieurs groupements, identiques ou différents choisis parmi R, OR, COR, COOR, NRR' (dans lesquels R et R' sont définis comme précédemment), nitro, cyano, ou atome d'halogène,
• par hétéroaryle on entend tout groupement mono ou bicyclique contenant 5 à 10 chaînons et pouvant contenir 1 à 3 hétéroatomes choisis parmi oxygène, soufre ou azote, ce groupement étant non substitué ou substitué par un ou plusieurs groupements, identiques ou différents choisis parmi R, OR, COR, COOR, NRR' (dans lesquels R et R' sont définis comme précédemment), nitro, cyano, ou atome d'halogène,
leurs énantiomères et diastéréoisomères ainsi que leurs sels d'addition à un acide ou à une base pharmaceutiquement acceptable.

2. Composés de formule (I) selon la revendication 1 pour lesquels B représente un groupement COOR, leurs énantiomères et diastéréoisomères, ainsi que leurs sels d'addition à un acide ou une base pharmaceutiquement acceptable.

3. Composés de formule (I) selon la revendication 2 pour lesquels B représente un groupement COOR où R représente un atome d'hydrogène, leurs énantiomères et diastéréoisomères, ainsi que leurs sels d'addition à un acide ou une base pharmaceutiquement acceptable.

4. Composés de formule (I) selon la revendication 2 pour lesquels B représente un groupement COOR où R représente un groupement alkyle(C₁-C₆)linéaire ou ramifié, leurs énantiomères et diastéréoisomères, ainsi que leurs sels d'addition à un acide ou une base pharmaceutiquement acceptable.

5. Composés de formule (I) selon la revendication 1 pour lesquels B représente un groupement alkyle(C₁-C₆)linéaire ou ramifié substitué par un groupement COOR, leurs énantiomères et diastéréoisomères, ainsi que leurs sels d'addition à un acide ou une base pharmaceutiquement acceptable.

6. Composés de formule (I) selon la revendication 1 pour lesquels B représente un groupement alkyle(C₁-C₆)linéaire ou ramifié substitué par un groupement OR, leurs énantiomères et diastéréoisomères, ainsi que leurs sels d'addition à un acide ou une base pharmaceutiquement acceptable.

7. Composés de formule (I) selon la revendication 1 pour lesquels G₁ représente un groupement -O-(CH₂)ₚ-O- dans lequel p est un entier tel que 0<p<6, leurs énantiomères et diastéréoisomères, ainsi que leurs sels d'addition à un acide ou une base pharmaceutiquement acceptable.

8. Composés de formule (I) selon la revendication 1 pour lesquels Cy représente un groupement naphtalène, leurs énantiomères et diastéréoisomères, ainsi que leurs sels d'addition à un acide ou une base pharmaceutiquement acceptable.

9. Composés de formule (I) selon la revendication 1 pour lesquels A représente un groupement NHCOR, leurs énantiomères et diastéréoisomères, ainsi que leurs sels d'addition à un acide ou une base pharmaceutiquement acceptable.

10. Composé de formule (I) selon la revendication 1 qui est le 4'-[4-({8-[2-(acétylamino)éthyl]-2-naphtyl}oxy)butoxy][1,1'-biphényl]-4-carboxylate de méthyle, ainsi que ses sels d'addition à un acide ou une base phannaceutiquement acceptable.

11. Composé de formule (I) selon la revendication 1 qui est l'acide 4'-[4-({8-[2-(acétylamino)éthyl]-2-naphtyl}oxy)butoxy][1,1'-biphényl]-4-carboxylique, ainsi que ses sels d'addition à un acide ou une base pharmaceutiquement acceptable.

12. Composé de formule (I) selon la revendication 1 qui est le *N*-{2-[7-(4-{[4'-(hydroxyméthyl)[1,1'-biphényl]-4-yl]oxy}butoxy)-1-naphtyl]éthyl}acétamide, ainsi que ses sels d'addition à un acide ou une base pharmaceutiquement acceptable.

13. Composé de formule (I) selon la revendication 1 qui est le *N*-(2-{7-[4-([1,1'-biphényl]-4-yloxy)butoxy]-1-naphtyl}éthyl)acétamide, ainsi que ses sels d'addition à un acide ou une base pharmaceutiquement acceptable.

14. Composé de formule (I) selon la revendication 1 qui est le *N*-(2-{7-[4-([1,1'-biphényl]-3-yloxy)butoxy]-1-naphtyl}éthyl)acétamide, ainsi que ses sels d'addition à un acide ou une base pharmaceutiquement acceptable.

15. Procédé de préparation des composés de formule (I) selon la revendication 1 **caractérisé en ce que** l'on utilise comme produit de départ le composé de formule (IV) :
MeO-Cy-G₂-A (IV)
dans laquelle A, G₂ et Cy sont tels que définis dans la formule (I),
que l'on soumet à une déméthylation en utilisant des agents classiques comme HBr, AlCl₃, AlBr₃, BBr₃ ou des systèmes binaires acide de Lewis / nucléophile comme AlCl₃ / PhCH₂SH ou BBr₃ / Me₂S par exemple, pour obtenir le composé de formule (V) :
HO-Cy-G₂-A (V)
dans laquelle A, G₂ et Cy sont définis comme précédemment,
qui est transformé, de façon classique,
- par action du *N*,*N*-diméthylthiocarbamate de sodium par exemple, en thiol correspondant de formule (VI) :
HS-Cy-G₂-A (VI)
dans laquelle A, G₂ et Cy sont tels que définis précédemment,
- ou en dérivé aminé correspondant de formule (VII) :
RNH-Cy-G₂-A (VII)
dans laquelle A, G₂, Cy et R sont définis comme précédemment
les composés de formule (V), (VI) et (VII) représentant le composé de formule (VIII) :
HX"-Cy-G₂-A (VIII)
dans laquelle Cy, G₂, X" et A sont tels que définis précédemment,
composé de formule (VIII) sur lequel on condense un composé de formule (IX) : 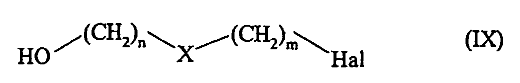 dans laquelle Hal représente un atome de brome, de chlore ou d'iode, et X, n et m sont tels que définis dans la formule (I), (étant entendu que l'on ne peut pas avoir deux hétéroatomes consécutifs et que la chaîne ainsi définie peut comporter une ou plusieurs insaturations),
pour conduire au composé de formule (X) :
HO-(CH₂)ₙ-X-(CH₂)ₘ-X"-Cy-G₂-A (X)
dans laquelle A, G₂, Cy, X, X", n et m sont définis comme précédemment (étant entendu que l'on ne peut pas avoir deux hétéroatomes consécutifs dans l'enchaînement HO-(CH₂)ₙ-X-(CH₂)ₘ-X"- et que la chaîne ainsi définie peut comporter une ou plusieurs insaturations),
dont la fonction hydroxyle est transformée classiquement en groupe partant comme par exemple un mésylate, un tosylate, ou un dérivé halogéné, pour conduire au composé de formule (X') :
E-(CH₂)ₙ-X-(CH₂)ₘ-X"-Cy-G₂-A (X')
dans laquelle A, G₂, Cy, X, X", n et m sont définis comme précédemment et E représente un groupement mésyle ou tosyle ou un atome d'halogène,
sur lequel on fait agir en milieu basique un composé de formule (XI) :
B'-Ph-Ph-X'H (XI)
dans laquelle X' est tel que défini dans la formule (I), et B' peut prendre les mêmes valeurs que B défini dans la formule (I) à l'exception des groupements COOH et alkyle substitué par un groupement COOH,
pour conduire au composé de formule (I/a), cas particulier des composés de formule (I) :
B'-Ph-Ph-G₁-Cy-G₂-A (I/a)
dans laquelle A, G₂, G₁, Cy et B' sont tels que définis précédemment,
composé de formule (I/a) étant soumis, lorsque B' représente un groupement COOR₁' ou alkyle substitué par un groupement COOR₁' (où R₁' peut prendre toutes les valeurs de R défini précédemment à l'exception de l'atome d'hydrogène), à une saponification pour conduire au composé de formule (I/b), cas particulier des composés de formule (I) :
B"-Ph-Ph-G₁-Cy-G₂-A (I/b)
dans laquelle A, G₂, G₁, et Cy sont tels que définis précédemment, et B" représente un groupement COOH ou alkyle substitué par un groupement COOH,
l'ensemble des composés (I/a) et (I/b) formant le composé de formule (I) qui peut être purifié si on le désire par une technique classique de purification, dont on sépare éventuellement les isomères selon une technique classique de séparation et que l'on transforme, le cas échéant, en leurs sels d'addition à un acide ou à une base pharmaceutiquement acceptable.

16. Compositions pharmaceutiques contenant comme principe actif au moins un composé de formule (I) selon l'une quelconque des revendications 1 à 14 ou un de leurs sels d'addition avec un acide ou une base pharmaceutiquement acceptables en combinaison avec un ou plusieurs excipients pharmaceutiquement acceptables.

17. Compositions pharmaceutiques selon la revendication 16 utiles pour la fabrication d'un médicament pour traiter les troubles liés au système mélatoninergique.

## Patentansprüche

1. Verbindungen der Formel (I): 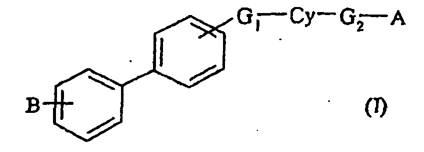 in der:
□ B ein Wasserstoffatom oder eine Gruppe COOR, CONRR' oder eine geradkettige oder verzweigte (C₁-C₆)-Alkylgruppe, die durch eine Gruppe COOR, CONRR' oder OR substituiert ist (worin R und R', die gleichartig oder verschieden sind, ein Wasserstoffatom oder eine geradkettige oder verzweigte (C₁-C₆)-Alkylgruppe, geradkettige oder verzweigte (C₂-C₆)-Alkenylgruppe, geradkettige oder verzweigte (C₂-C₆)-Alkinylgruppe, Arylgruppe, geradkettige oder verzweigte Aryl-(C₁-C₆)-alkylgruppe, Heteroarylgruppe, geradkettige oder verzweigte Heteroaryl-(C₁-C₆)-alkylgruppe, geradkettige oder verzweigte Polyhalogen-(C₁-C₆)-alkylgruppe, (C₃-C₈)-Cycloalkylgruppe oder geradkettige oder verzweigte (C₃-C₈)-Cycloalkyl-(C₁-C₆)-alkylgruppe bedeuten, wobei R und R' gemeinsam mit dem sie tragenden Stickstoffatom eine Morpholinyl-, Piperidinyl-, Piperazinyl- oder Pyrrolidinylgruppe bilden können) bedeutet,
□ G₁ eine Kette -X'-(CH₂)ₙ-X-(CH₂)ₘ-X" bedeutet, in der
◆ X ein Sauerstoff- oder Schwefelatom oder eine Gruppe CH₂ oder NR (worin R die oben angegebenen Bedeutungen besitzt) bedeutet,
◆ X' und X", die gleichartig oder verschieden sind, ein Sauerstoff- oder Schwefelatom oder eine Gruppe NR (worin R die oben angegebenen Bedeutungen besitzt) bedeuten,
◆ n und m, die gleichartig oder verschieden sind, 0, 1, 2, 3, 4 oder 5 darstellen,
mit der Maßgabe, daß nicht zwei aufeinanderfolgende Heteroatome vorliegen und die in dieser Weise definierte Kette eine oder mehrere Unsättigungen aufweisen kann,
□ Cy eine Gruppe der Formel (II) 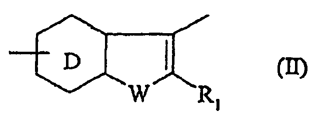 in der D Phenyl oder Pyridin, W ein Sauerstoff- oder Schwefelatom oder eine Gruppe CH₂ oder NR (worin R die oben angegebenen Bedeutungen besitzt), R₁ ein Halogenatom oder eine Gruppe R, OR oder COOR (worin R die oben angegebenen Bedeutungen besitzt) bedeuten und das Symbol 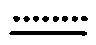 bedeutet, daß die Bindung einfach oder doppelt ist, mit der Maßgabe, daß die Wertigkeit der Atome berücksichtigt ist,
oder eine Gruppe der Formel (III) 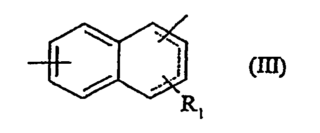 in der R₁ und das Symbol 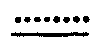 die oben angegebenen Bedeutungen besitzen, darstellt,
□ G₂ eine Kette bedeutet, die 1 bis 6 Kohlenstoffatome aufweist und gegebenenfalls durch eine oder mehrere Gruppen ausgewählt aus R, OR, COR, COOR (worin R die oben angegebenen Bedeutungen besitzt) oder Halogenatome substituiert ist,
□ und A eine Gruppe NRCOR', NRCSR', CONRR', CSNRR', NRCONR'R" oder NRCSNR'R" (worin R und R' die oben angegebenen Bedeutungen besitzen und R" die gleichen Bedeutungen besitzen kann wie R und R') darstellt,
mit der Maßgabe, daß man:
• unter Aryl eine Phenyl- oder Naphthylgruppe versteht, die nicht substituiert oder durch eine oder mehrere gleichartige oder verschiedenartige Gruppen ausgewählt aus R, OR, COR, COOR, NRR' (worin R und R' die oben angegebenen Bedeutungen besitzen), Nitro, Cyano und Halogenatomen substituiert ist,
• unter Heteroaryl jede mono- oder bicyclische Gruppe, die 5 bis 10 Kettenglieder aufweist und 1 bis 3 Heteroatome ausgewählt aus Sauerstoff, Schwefel oder Stickstoff enthält, versteht, wobei diese Gruppe nicht substituiert ist oder durch eine oder mehrere gleichartige oder verschiedenartige Gruppen ausgewählt aus R, OR, COR, COOR, NRR' (worin R und R' die oben angegebenen Bedeutungen besitzen), Nitro, Cyano und Halogenatomen substituiert ist,
deren Enantiomere und Diastereoisomere sowie deren Additionssalze mit einer pharmazeutisch annehmbaren Säure oder Base.

2. Verbindungen der Formel (I) nach Anspruch 1, worin B eine Gruppe COOR darstellt, deren Enantiomere und Diastereoisomere sowie deren Additionssalze mit einer pharmazeutisch annehmbaren Säure oder Base.

3. Verbindungen der Formel (I) nach Anspruch 2, worin B eine Gruppe COOR bedeutet, worin R ein Wasserstoffatom darstellt, deren Enantiomere und Diastereoisomere sowie deren Additionssalze mit einer pharmazeutisch annehmbaren Säure oder Base.

4. Verbindungen der Formel (I) nach Anspruch 2, worin B eine Gruppe COOR bedeutet, worin R eine geradkettige oder verzweigte (C₁-C₆)-Alkylgruppe darstellt, deren Enantiomere und Diastereoisomere sowie deren Additionssalze mit einer pharmazeutisch annehmbaren Säure oder Base.

5. Verbindungen der Formel (I) nach Anspruch 1, worin B eine geradkettige oder verzweigte (C₁-C₆)-Alkylgruppe, die durch eine Gruppe COOR substituiert ist, bedeutet, deren Enantiomere und Diastereoisomere sowie deren Additionssalze mit einer pharmazeutisch annehmbaren Säure oder Base.

6. Verbindungen der Formel (I) nach Anspruch 1, worin B eine geradkettige oder verzweigte (C₁-C₆)-Alkylgruppe, die durch eine Gruppe OR substituiert ist, bedeutet, deren Enantiomere und Diastereoisomere sowie deren Additionssalze mit einer pharmazeutisch annehmbaren Säure oder Base.

7. Verbindungen der Formel (I) nach Anspruch 1, worin G₁ eine Gruppe -O-(CH₂)ₚ-O- bedeutet, in der p eine ganze Zahl mit einem Wert bedeutet, daß die Beziehung 0 < p < 6 erfüllt ist, deren Enantiomere und Diastereoisomere sowie deren Additionssalze mit einer pharmazeutisch annehmbaren Säure oder Base.

8. Verbindungen der Formel (I) nach Anspruch 1, worin Cy eine Naphthalingruppe darstellt, deren Enantiomere und Diastereoisomere sowie deren Additionssalze mit einer pharmazeutisch annehmbaren Säure oder Base.

9. Verbindungen der Formel (I) nach Anspruch 1, worin A eine Gruppe NHCOR bedeutet, deren Enantiomere und Diastereoisomere sowie deren Additionssalze mit einer pharmazeutisch annehmbaren Säure oder Base.

10. Verbindung der Formel (I) nach Anspruch 1, nämlich 4'-[4-({8-[2-(Acetylamino)-ethyl]-2-naphthyl}-oxy)-butoxy][1,1'-biphenyl]-4-carbonsäuremethylester sowie dessen Additionssalze mit einer pharmazeutisch annehmbaren Säure oder Base.

11. Verbindung der Formel (I) nach Anspruch 1, nämlich 4'-[4-({8-[2-(Acetylamino)-ethyl]-2-naphthyl}-oxy)-butoxy][1,1'-biphenyl)-4-carbonsäure sowie deren Additionssalze mit einer pharmazeutisch annehmbaren Säure oder Base.

12. Verbindung der Formel (I) nach Anspruch 1, nämlich *N*-{2-[7-(4-{[4'-(Hydroxymethyl)[1,1'-biphenyl]-4-yl]-oxy}-butoxy)-1-naphthyl]-ethyl}-acetamid sowie dessen Additionssalze mit einer pharmazeutisch annehmbaren Säure oder Base.

13. Verbindung der Formel (I) nach Anspruch 1, nämlich *N*-(2-{7-[4-([1,1'-Biphenyl]-4-yloxy)-butoxyl-1-naphthyl}-ethyl)-acetamid sowie dessen Additionssalze mit einer pharmazeutisch annehmbaren Säure oder Base.

14. Verbindung der Formel (I) nach Anspruch 1, nämlich *N*-(2-{7-[4-([1,1'-Biphenyl]-3-yloxy)-butoxy]-1-naphthyl}-ethyl)-acetamid sowie dessen Additionssalze mit einer pharmazeutisch annehmbaren Säure oder Base.

15. Verfahren zur Herstellung der Verbindungen der Formel (I) nach Anspruch 1, **dadurch gekennzeichnet, daß** man als Ausgangsprodukt die Verbindung der Formel (IV) verwendet:
MeO - Cy - G₁ - A (IV)
in der A, G₂ und Cy die bezüglich der Formel (I) angegebenen Bedeutungen besitzen,
welche man einer Demethylierung unterwirft unter Verwendung klassischer Mittel, wie HBr, AlCl₃, AlBr₃, BBr₃ oder binärer Lewissäure / Nucleophil-Systeme, wie beispielsweise AlCl₃ / PhCH₂SH oder BBr₃ / Me₂S, zur Bildung der Verbindung der Formel (V):
HO - Cy - G₂ - A (V)
in der A, G₂ und Cy die oben angegebenen Bedeutungen besitzen, welche man in klassischer Weise
- durch Einwirkung von beispielsweise Natrium-*N*,*N*-dimethylthiocarbamat in das entsprechende Thiol der Formel (VI):
HS - Cy G₂ - A (VI)
in der A, G₂ und Cy die oben angegebenen Bedeutungen besitzen,
- oder in das entsprechende Aminderivat der Formel (VII):
RNH - Cy - G₂ - A (VII)
in der A, G₂, Cy und R die oben angegebenen Bedeutungen besitzen, umwandelt,
wobei die Verbindungen der Formeln (V), (VI) und (VII) die Verbindung der Formel (VIII) bilden:
HX" - Cy - G₂ - A (VIII)
in der Cy, G₂, X" und A die oben angegebenen Bedeutungen besitzen,
welche Verbindung der Formel (VIII) man mit einer Verbindung der Formel (IX) kondensiert: 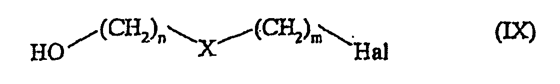 in der Hal ein Brom-, Chlor- oder Iodatom bedeutet und X, n und m die bezüglich der Formel (I) angegebenen Bedeutungen besitzen (mit der Maßgabe, daß nicht zwei aufeinanderfolgende Heteroatome vorliegen und die in dieser Weise definierte Kette eine oder mehrere Unsättigungen aufweisen kann),
zur Bildung der Verbindung der Formel (X):
HO - (CH₂)ₙ - X - (CH₂)ₘ - X" - Cy - G₂ - A (X)
in der A, G₂, Cy, X, X", n und m die oben angegebenen Bedeutungen besitzen (mit der Maßgabe, daß in der Kette HO - (CH₂)ₙ - X - (CH₂)ₘ - X" nicht zwei aufeinanderfolgende Heteroatome vorliegen und die in dieser Weise definierte Kette eine oder mehrere Unsättigungen aufweisen kann),
deren Hydroxylfunktion in klassischer Weise in eine austretende Gruppe umgewandelt wird, beispielsweise eine Mesylat- oder Tosylatgruppe oder ein Halogenderivat zur Bildung der Verbindung der Formel (X'):
E - (CH₂)ₙ - X - (CH₂)ₘ - X" - Cy - G₂ - A (X')
in der A, G₂, Cy, X, X", n und m die oben angegebenen Bedeutungen besitzen und E eine Mesyl- oder Tosylgruppe oder ein Halogenatom darstellt,
welche man in basischem Medium mit einer Verbindung der Formel (XI) umsetzt:
B' - Ph - Ph - X'H (XI)
in der X' die bezüglich der Formel (I) angegebenen Bedeutungen besitzt und B' die gleichen Bedeutungen aufweisen kann wie B, wie es für die Formel (I) definiert ist, mit Ausnahme der Gruppen COOH und der durch eine COOH-Gruppe substituierte Alkylgruppe,
zur Bildung der Verbindung der Formel (I/a), einem Sonderfall der Verbindungen der Formel (I):
B' - Ph - Ph - G₁ - Cy - G₂ - A (I/a)
in der A, G₂, G₁, Cy und B' die oben angegebenen Bedeutungen besitzen,
welche Verbindung der Formel (I/a), wenn B' eine Gruppe COOR₁' oder eine durch eine Gruppe COOR₁' substituierte Alkylgruppe bedeutet (worin R₁' sämtliche oben definierten Bedeutungen von R mit Ausnahme des Wasserstoffatoms aufweisen kann) man einer Verseifung unterwirft zur Bildung der Verbindung der Formel (I/b), einem Sonderfall der Verbindungen der Formel (I):
B" - Ph - Ph - G₁ - Cy - G₂ - A (I/b)
in der A, G₂, G₁ und Cy die oben angegebenen Bedeutungen besitzen und B" eine Gruppe COOH oder eine durch eine Gruppe COOH substituierte Alkylgruppe bedeutet,
wobei die Gesamtheit der Verbindungen (I/a) und (I/b), welche die Verbindungen der Formel (I) bilden, gewünschtenfalls mit Hilfe einer klassischen Reinigungsmethode gereinigt werden können, welche man gegebenenfalls mit Hilfe einer klassischen Trennmethode in ihre Isomeren auftrennt und welche man gegebenenfalls in ihre Additionssalze mit einer pharmazeutisch annehmbaren Säure oder Base überführt.

16. Pharmazeutische Zubereitungen, enthaltend als Wirkstoff mindestens eine Verbindung der Formel (I) nach einem der Ansprüche 1 bis 14 oder eines ihrer Additionssalze mit einer pharmazeutisch annehmbaren Säure oder Base in Kombination mit einem oder mehreren pharmazeutisch annehmbaren Trägermaterialien.

17. Pharmazeutische Zubereitungen nach Anspruch 16, nützlich für die Herstellung eines Arzneimittels zur Behandlung von Störungen, die mit dem melatoninergischen System verbunden sind.

## Claims

1. Compounds of formula (I) : 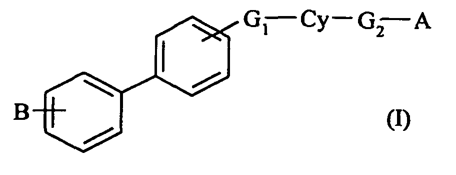 wherein :
□ B represents a hydrogen atom, a COOR group, a CONRR' group, or a linear or branched (C₁-C₆)alkyl group substituted by a COOR, CONRR' or OR group (wherein R and R', which may be identical or different, each represents a hydrogen atom, or a group linear or branched (C₁-C₆)alkyl, linear or branched (C₂-C₆)alkenyl, linear or branched (C₂-C₆)alkynyl, aryl, aryl-(C₁-C₆)alkyl in which the alkyl moiety may be linear or branched, heteroaryl, heteroaryl-(C₁-C₆)alkyl in which the alkyl moiety may be linear or branched, polyhalo-(C₁-C₆)alkyl in which the alkyl moiety may be linear or branched, (C₃-C₈)cycloalkyl or (C₃-C₈)cycloalkyl-(C₁-C₆)alkyl in which the alkyl moiety may be linear or branched, it being possible in addition for R and R' to form, together with the nitrogen atom carrying them, a morpholinyl, piperidyl, piperazinyl or pyrrolidinyl group),
□ G₁ represents a -X'-(CH₂)ₙ-X-(CH₂)ₘ-X"- chain wherein
◆ X represents an oxygen or sulphur atom, or a CH₂ or NR group (wherein R is as defined hereinbefore),
◆ X' and X", which may be identical or different, each represents an oxygen or sulphur atom or an NR group (wherein R is as defined hereinbefore),
◆ n and m, which may be identical or different, each represents 0, 1, 2, 3, 4 or 5, it being understood that it is not possible to have two consecutive hetero atoms and that the chain so defined may contain one or more unsaturations,
□ Cy represents a grouping of formula (II) 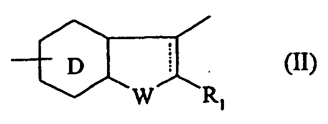 wherein D represents a phenyl or a pyridine, W represents an oxygen or sulphur atom, or a CH₂ or NR group (wherein R is as defined hereinbefore), R₁ represents a halogen atom or an R, OR or COOR group (wherein R is as defined hereinbefore) and the representation - - - denotes that the bond is single or double, it being understood that the valency of the atoms is respected,
or a grouping of formula (III) 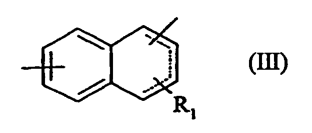 wherein R₁ and the representation - - - are as defined hereinbefore,
□ G₂ represents a chain containing from 1 to 6 carbon atoms that is optionally substituted by one or more groups selected from R, OR, COR, COOR (wherein R is as defined hereinbefore) and halogen atoms,
□ and A represents a NRCOR', NRCSR', CONRR', CSNRR', NRCONR'R" or NRCSNR'R" group (wherein R and R' are as defined hereinbefore and R" may have the same meanings as R and R'),
wherein:
• "aryl" is to be understood as meaning a phenyl or naphthyl group that is unsubstituted or substituted by one or more identical or different groups selected from R, OR, COR, COOR, NRR' (wherein R and R' are as defined hereinbefore), nitro, cyano and halogen atoms,
• "heteroaryl" is to be understood as meaning any mono- or bi-cyclic group having from 5 to 10 ring members and capable of containing from 1 to 3 hetero atoms selected from oxygen, sulphur and nitrogen, that group being unsubstituted or substituted by one or more identical or different groups selected from R, OR, COR, COOR, NRR' (wherein R and R' are as defined hereinbefore), nitro, cyano and halogen atoms,
their enantiomers and diastereoisomers, and also addition salts thereof with a pharmaceutically acceptable acid or base.

2. Compounds of formula (I) according to claim 1 wherein B represents a COOR group, their enantiomers and diastereoisomers, and also addition salts thereof with a pharmaceutically acceptable acid or base.

3. Compounds of formula (I) according to claim 2 wherein B represents a COOR group in which R represents a hydrogen atom, their enantiomers and diastereoisomers, and also addition salts thereof with a pharmaceutically acceptable acid or base.

4. Compounds of formula (I) according to claim 2 wherein B represents a COOR group in which R represents a linear or branched (C₁-C₆)alkyl group, their enantiomers and diastereoisomers, and also addition salts thereof with a pharmaceutically acceptable acid or base.

5. Compounds of formula (I) according to claim 1 wherein B represents a linear or branched (C₁-C₆)alkyl group substituted by a COOR group, their enantiomers and diastereoisomers, and also addition salts thereof with a pharmaceutically acceptable acid or base.

6. Compounds of formula (I) according to claim 1 wherein B represents a linear or branched (C₁-C₆)alkyl group substituted by an OR group, their enantiomers and diastereoisomers, and also addition salts thereof with a pharmaceutically acceptable acid or base.

7. Compounds of formula (I) according to claim 1 wherein G₁ represents a -O-(CH₂)ₚ-O-group in which p is an integer such that 0<p<6, their enantiomers and diastereoisomers, and also addition salts thereof with a pharmaceutically acceptable acid or base.

8. Compounds of formula (I) according to claim 1 wherein Cy represents a naphthalene group, their enantiomers and diastereoisomers, and also addition salts thereof with a pharmaceutically acceptable acid or base.

9. Compounds of formula (I) according to claim 1 wherein A represents a NHCOR group, their enantiomers and diastereoisomers, and also addition salts thereof with a pharmaceutically acceptable acid or base.

10. Compound of formula (I) according to claim 1 which is methyl 4'-[4-({8-[2-(acetylamino)ethyl]-2-naphthyl}oxy)butoxy]-[1,1'-biphenyl]-4-carboxylate, and addition salts thereof with a pharmaceutically acceptable acid or base.

11. Compound of formula (I) according to claim 1 which is 4'-[4-({8-[2-(acetylamino)ethyl]-2-naphthyl}oxy)butoxy]-[1,1'-biphenyl]-4-carboxylic acid, and addition salts thereof with a pharmaceutically acceptable acid or base.

12. Compound of formula (I) according to claim 1 which is *N*-{2-[7-(4-{[4'-(hydroxymethyl)-[1,1'-biphenyl]-4-yl]oxy}butoxy)-1-naphthyl]ethyl}acetamide, and addition salts thereof with a pharmaceutically acceptable acid or base.

13. Compound of formula (I) according to claim 1 which is *N*-(2-{7-[4-([1,1'-biphenyl]-4-yloxy)butoxy]-1-naphthyl}ethyl)acetamide, and addition salts thereof with a pharmaceutically acceptable acid or base.

14. Compound of formula (I) according to claim 1 which is *N*-(2-{7-[4-([1,1'-biphenyl]-3-yloxy)butoxy]-1-naphthyl}ethyl)acetamide, and addition salts thereof with a pharmaceutically acceptable acid or base.

15. Process for the preparation of compounds of formula (I) according to claim 1, **characterised in that** there is used as starting material a compound of formula (IV) :
MeO-Cy-G₂-A (IV)
wherein A, G₂ and Cy are as defined for formula (I),
which is subjected to demethylation using conventional agents, such as HBr, AlCl₃, AlBr₃, BBr₃ or Lewis acid/nucleophile binary systems such as AlCl₃/PhCH₂SH or BBr₃/Me₂S, for example, to obtain a compound of formula (V) :
HO-Cy-G₂-A (V)
wherein A, G₂ and Cy are as defined hereinbefore,
which is converted in conventional manner
- by the action of sodium *N*,*N*-dimethylthiocarbamate for example, to the corresponding thiol of formula (VI) :
HS-Cy-G₂-A (VI)
wherein A, G₂ and Cy are as defined hereinbefore,
- or to the corresponding amine compound of formula (VII) :
RNH-Cy-G₂-A (VII)
wherein A, G₂, Cy and R are as defined hereinbefore,
the compounds of formula (V), (VI) and (VII) representing the compound of formula (VIII) :
HX"-Cy-G₂-A (VIII)
wherein Cy, G₂, X" and A are as defined hereinbefore,
which compound of formula (VIII) is condensed with a compound of formula (IX) : 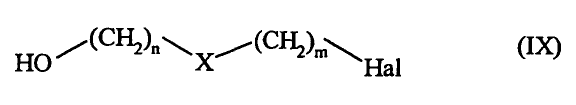 wherein Hal represents a bromine, chlorine or iodine atom, and X, n and m are as defined for formula (I), (it being understood that it is not possible to have two consecutive hetero atoms and that the chain so defined may contain one or more unsaturations),
to yield a compound of formula (X) :
HO-(CH₂)ₙ-X-(CH₂)ₘ-X"-Cy-G₂-A (X)
wherein A, G₂, Cy, X, X", n and m are as defined hereinbefore (it being understood that it is not possible to have two consecutive hetero atoms in the HO-(CH₂)ₙ-X-(CH₂)ₘ-X"-chain and that the chain so defined may contain one or more unsaturations),
the hydroxy function of which is converted in conventional manner into a leaving group, such as, for example, a mesylate, a tosylate, or a halogenated compound, to yield a compound of formula (X') :
E-(CH₂)ₙ-X-(CH₂)ₘ-X"-Cy-G₂-A (X')
wherein A, G₂, Cy, X, X", n and m are as defined hereinbefore and E represents a mesyl or tosyl group or a halogen atom,
which is reacted in basic medium with a compound of formula (XI) :
B'-Ph-Ph-X'H (XI)
wherein X' is as defined for formula (I), and B' may have the same meanings as B as defined for formula (I) with the exception of the groups COOH and alkyl substituted by a COOH group,
to yield a compound of formula (I/a), a particular case of the compounds of formula (I) :
B'-Ph-Ph-G₁-Cy-G₂-A (I/a)
wherein A, G₂, G₁, Cy and B' are as defined hereinbefore,
the compound of formula (I/a) being subjected, when B' represents a COOR₁' group or alkyl substituted by a COOR₁' group (wherein R₁' may have any of the meanings of R defined hereinbefore with the exception of a hydrogen atom), to hydrolysis to yield a compound of formula (I/b), a particular case of the compounds of formula (I) :
B"-Ph-Ph-G₁-Cy-G₂-A (I/b)
wherein A, G₂, G₁ and Cy are as defined hereinbefore, and B" represents a COOH group or alkyl substituted by a COOH group,
the totality of the compounds (I/a) and (I/b) constituting the compounds of formula (I) which may, if desired, be purified by a conventional purification technique, are optionally separated into their isomers according to a conventional separation technique and, if desired, are converted into addition salts with a pharmaceutically acceptable acid or base.

16. Pharmaceutical compositions comprising as active ingredient at least one compound of formula (I) according to any one of claims 1 to 14 or a pharmaceutically acceptable addition salt thereof with an acid or a base in combination with one or more pharmaceutically acceptable excipients.

17. Pharmaceutical compositions according to claim 16 for use in the production of a medicament to treat disorders associated with the melatoninergic system.
